(19) Europäisches Patentamt
European Patent Office
Office européen des brevets

(11) **EP 3 019 076 B1**

(12) **EUROPEAN PATENT SPECIFICATION**

(45) Date of publication and mention
of the grant of the patent:
**17.03.2021 Bulletin 2021/11**

(21) Application number: **14823539.3**

(22) Date of filing: **07.07.2014**

(51) Int Cl.:
*A61B 5/02* (2006.01)          *A61B 5/0215* (2006.01)
*A61B 5/024* (2006.01)          *A61B 5/029* (2006.01)
*A61B 5/22* (2006.01)

(86) International application number:
**PCT/US2014/045538**

(87) International publication number:
**WO 2015/006191 (15.01.2015 Gazette 2015/02)**

(54) **ARTERIAL PRESSURE-BASED DETERMINATION OF CARDIOVASCULAR PARAMETERS**

AUF ARTERIELLEM BLUTDRUCK BASIERENDE BESTIMMUNG VON KARDIOVASKULÄREN PARAMETERN

DÉTERMINATION DE PARAMÈTRES CARDIOVASCULAIRES SUR LA BASE DE LA PRESSION ARTÉRIELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.07.2013 US 201361843743 P**

(43) Date of publication of application:
**18.05.2016 Bulletin 2016/20**

(73) Proprietor: **Edwards Lifesciences Corporation Irvine, CA 92614 (US)**

(72) Inventor: **JIAN, Zhongping Irvine, CA 92614 (US)**

(74) Representative: **Eisenführ Speiser Patentanwälte Rechtsanwälte PartGmbB Postfach 31 02 60 80102 München (DE)**

(56) References cited:
**EP-B1- 0 947 941          KR-B1- 100 821 409
US-A1- 2008 287 812          US-A1- 2009 062 666
US-A1- 2010 016 739          US-A1- 2011 275 943**

• LANGEWOUTERS G J ET AL: "The static elastic properties of 45 human thoracic and 20 abdominal aortas in vitro and the parameters of a new model", JOURNAL OF BIOMECHANICS, PERGAMON PRESS, NEW YORK, NY, US, vol. 17, no. 6, 1 January 1984 (1984-01-01), pages 425-435, XP022917641, ISSN: 0021-9290, DOI: 10.1016/0021-9290(84)90034-4 [retrieved on 1984-01-01]

**Description**

BACKGROUND

Indicators such as stroke volume (SV), cardiac output (CO), end-diastolic volume, ejection fraction, stroke volume variation (SVV), pulse pressure variation (PPV), and systolic pressure variations (SPV), among others, are important not only for diagnosis of disease, but also for "real-time" monitoring of preload dependence, fluid responsiveness, or volume responsiveness conditions of both human and animal subjects. Few hospitals are therefore without some form of equipment to monitor one or more of these cardiac parameters. Many techniques, including invasive techniques, non-invasive techniques, and combinations of the two, are in use and even more have been proposed in the literature.

Many of the techniques used to measure SV can be adapted to provide an estimate of CO as well, because CO is generally defined as SV times the subject's heart rate (HR), which is usually available to monitoring equipment. Conversely, most devices that estimate CO also estimate SV in their calculations. One way to estimate SVV is simply to collect multiple SV values and calculate the differences from measurement interval to measurement interval.

US 2011/0275943 A1 relates to an arterial pressure-based, automatic publication classification determination of a cardiovascular parameter.

FIELD OF TECHNOLOGY

This disclosure is related to the field of patient hemodynamic monitoring and digital signal processing. This disclosure specifically relates to a method of calculating real-time cardiovascular parameters including but not limited to CO, SV, and vascular resistance.

BRIEF SUMMARY

Methods are described in this document for determining a cardiovascular parameter of a subject. Such methods may include steps including operating a processing system to receive an input signal that corresponds to arterial blood pressure, operating the processing system to calculate a first statistical moment of numerical values corresponding to a sequence of measured arterial pressure values, operating the processing system to calculate the cardiovascular parameter as a function of the first statistical moment and a vascular tone factor, and operating a display to present an indication of the calculated cardiovascular parameter to a user.

The invention provides a method as set forth in independent claim 1, and an apparatus as set forth in independent claim 9. The dependent claims are directed to further developments of the invention.

In some methods the first scaling factor is inversely proportional to a mean of a measured arterial pressure of the subject, inversely proportional to a mean of a measured arterial pressure of the subject minus a constant, inversely proportional to at least one of the subject's measured systolic or diastolic pressure, or based on at least one of the subject's race, age, gender, or body surface area.

In some methods the cardiovascular parameter is a cardiac stroke volume, and the method may further include determining the subject's heart rate and calculating the subject's cardiac output as the multiplication product of the stroke volume and the heart rate.

In some methods operating the processing system to calculate the first statistical moment of numerical values corresponding to the sequence of measured arterial pressure values includes calculating a standard deviation of the numerical values.

In some methods operating the processing system to calculate the second scaling factor as the function of the second statistical moment of the set of numerical values corresponding to the sequence of measured arterial pressure values includes calculating a standard deviation of the numerical values.

In some methods operating the processing system to calculate a first statistical moment of numerical values corresponding to a sequence of measured arterial pressure values includes operating the processing system to digitize an analog pressure signal to produce a sequence of digital values each of which corresponds to a arterial pressure value measured at a predetermined time, operating the processing system to form a pressure-weighted sequence of values comprising counts of measured arterial pressure values within selected pressure value windows, and then operating the processing system to calculate the first statistical moment of the pressure-weighted sequence of values.

In some embodiments, a method is provided for determining a cardiac output. The method further comprises: receiving, using a computing device processor, blood pressure data; determining, using a computing device processor, a standard deviation associated with the blood pressure data; determining, using a computing device processor, a pulse rate associated with the blood pressure data; determining, using a computing device processor, a compliance factor associated with the blood pressure data; determining, using a computing device processor, a function associated with the blood pressure waveform data; and determining, using a computing device processor, the cardiac output based on the standard deviation, the pulse rate, the compliance factor, and the function.

In some embodiments, the compliance factor is inversely proportional to a mean of an arterial pressure.

In some embodiments, the compliance factor is inversely proportional to a mean of an arterial pressure less a constant.

In some embodiments, the compliance factor is inversely proportional to at least one of a systolic pressure or a diastolic pressure.

In some embodiments, the compliance factor is based on at least one of a race, an age, a gender, or a body surface area of a person.

In some embodiments, the function comprises an arterial compliance function.

According to some embodiments of the invention, the apparatus further comprises a memory; a processor; and a module stored in the memory, executable by the processor, and configured to perform any method described herein. For example the module may be further configured to: receive blood pressure data; determine a standard deviation of the blood pressure data; determine a pulse rate associated with the blood pressure data; determine a compliance factor associated with the blood pressure data; determine a function associated with the blood pressure data; and determine the cardiac output based on the standard deviation, the pulse rate, the compliance factor, and the function.

In some embodiments, the apparatus further comprises at least one of a catheter and a disposable pressure transducer associated with the catheter.

In some embodiments, the apparatus further comprises at least one of a signal filter or an AC/DC converter.

In some embodiments, a computer program product is provided for determining a cardiac output. The computer program product comprises a non-transitory computer-readable medium comprising a set of codes for causing a computer to: receive blood pressure data; determine a standard deviation of the blood pressure data; determine a pulse rate associated with the blood pressure data; determine a compliance factor associated with the blood pressure data; determine a function associated with the blood pressure data; and determine the cardiac output based on the standard deviation, the pulse rate, the compliance factor, and the function.

In some embodiments, an apparatus is provided for determining a cardiac output. The apparatus further comprises means for receiving blood pressure data; means for determining a standard deviation associated with the blood pressure data; means for determining a pulse rate associated with the blood pressure data; means for determining a compliance factor associated with the blood pressure data; means for determining a function associated with the blood pressure data; and means for determining the cardiac output based on the standard deviation, the pulse rate, the compliance factor, and the function.

BRIEF DESCRIPTION OF THE DRAWINGS

Having thus described embodiments of the disclosure in general terms, reference will now be made to the accompanying drawings, where:

> Figure 1 illustrates a measured blood pressure waveform over a single cardiac cycle, during which the blood pressure ranges between a diastolic pressure $P_{dia}$ and a systolic pressure $P_{sys}$;
> Figure 2 illustrates the digitization of the analog blood pressure waveform of Fig. 1 into a sequence of digital values.
> Figure 3 illustrates a process flow for a method for determining cardiac output; and
> Figure 4 shows an exemplary apparatus for implementing the various methods described herein.

DETAILED DESCRIPTION OF EMBODIMENTS OF THE DISCLOSURE

Some embodiments of the present disclosure are described more fully below with reference to the accompanying drawings. The invention described here may be embodied in many different forms and should not be construed as limited to the specific embodiments included in this document. The embodiments described here are provided so that this disclosure may satisfy applicable legal requirements in disclosing examples embodying the invention. The scope of the invention and the legal protections conferred should thus be ascertained primarily by reference to the appended claims.

One way to measure SV or CO is to mount a flow-measuring device on a catheter, and position the device in or near the subject's heart. Some such devices inject either a bolus of material or energy (usually heat) at an upstream position, such as in the right atrium, and determine flow based on the characteristics of the injected material or energy sensed at a downstream position, such as in the pulmonary artery. Patents that disclose implementations of such invasive techniques (in particular, thermodiludon) include: U.S. Pat. No. 4,236,527 (Newbower et al., 2 Dec. 1980); U.S. Pat. No. 4,507,974 (Yelderman, 2 Apr. 1985); U.S. Pat. No. 5,146,414 (McKown, et al., 8 Sep. 1992); and U.S. Pat. No. 5,687,733 (McKown, et al., 18 Nov. 1997). Other invasive devices are based on the known Fick technique, according to which CO is calculated as a function of oxygenation of arterial and mixed venous blood. Invasive techniques have obvious disadvantages, especially when the subjects in need of such monitoring are already in the hospital due to a serious condition. Invasive methods also have less obvious disadvantages. For example, some techniques such as thermodilution rely on assumptions, such as uniform dispersion of the injected heat, which can affect the accuracy of the measurements. Moreover, the introduction of an instrument into the blood flow may affect the value that the instrument measures. Doppler techniques, using invasive as well as non-invasive transducers, have also been used to obtain flow rate data that can then be used to calculate SV and CO. However, these systems are typically expensive, and their accuracy depends on precise knowledge of the diameter and general geometry of the flow channel. Such precise knowledge is, however, seldom possible, especially under conditions where real-time monitoring is desired.

One blood flow characteristic that can be obtained with minimal or no invasion is blood pressure. In addition to causing minimal patient trauma, blood pressure measurement technology has the added benefit of being accurate and continuous. Many systems rely on the pulse contour method (PCM), which calculates an estimate of one or more cardiac parameters of interest, such as CO, from characteristics of a blood pressure waveform. In the PCM, "Windkessel" parameters, such as characteristic impedance of the aorta, compliance, and total peripheral resistance, are often used to construct a linear or non- linear hemodynamic model of the aorta. In essence, blood flow is analogized to a flow of electrical current in a circuit in which an impedance is in series with a parallel-connected resistance and capacitance (compliance). The three required parameters of the model are usually determined either empirically, through a complex calibration process, or from compiled "anthropometric" data, i.e., data about the age, sex, height, weight, and/or other parameters of other patients or test subjects. U.S. Pat. No. 5,400,793 (Wesseling, 28 Mar. 1995) and U.S. Pat. No. 5,535,753 (Petrucelli et al., 16 Jul. 1996) disclose systems that rely on a Windkessel circuit model to determine CO. PCM-based systems can monitor SV-derived cardiac parameters using blood pressure measurements taken using a variety of measurement apparatus, such as a finger cuff, and can do so more or less continuously.

This disclosure provides a method to calculate cardiovascular parameters such as CO and SV based on blood pressure waveforms. In today's patient hemodynamic monitoring, blood pressure is routinely monitored and many parameters that are of great clinical use, such as CO, SV, and SVV can be derived from blood pressure waveforms and displayed by monitors. Accurately calculating those parameters is of great importance because it can help clinicians know more about patients and make more informed decisions in treating them.

In a first method, a subject's cardiac output CO can be calculated as $CO = K \times \sigma \times PR$, where $\sigma$ is the standard deviation of a set of values corresponding to or derived from a blood pressure waveform, PR is the pulse rate derived from the blood pressure waveform, and K is a function of blood pressure waveform characteristic parameters that accounts for the effect of vascular tone. As used herein, a blood pressure waveform is associated with blood pressure data. Methods for computing CO and other cardiovascular parameters are described, for example, in United States Patent Nos. 7,967,757, and 8,721,556, in International Publication No. WO2010/091055, and in other published documents.

The present disclosure proposes a method for calculating cardiac output as a function of waveform characteristic parameters and a compliance factor, *i.e.*,

$$CO = C \times K \times \sigma \times PR$$

where PR is the subject's pulse rate derived from the blood pressure waveform, and $\sigma$ is the standard deviation of values taken from or otherwise corresponding to or indicative of the blood pressure waveform. C is a factor related to the subject's vascular tone, which relates to the compliance of the subject's vasculature, which may be a function of arterial pressure and updated in real-time (or substantially real-time). K is a function of waveform characteristic parameters.

Compliance, which is defined as a change in blood vessel volume divided by a change in blood pressure, generally decreases at higher pressure. To model the relevant part of the overall vascular tone or compliance function, C may be treated as inversely proportional to the arterial pressure, linearly or nonlinearly. As an example, C can be equal to (1/MAP), where MAP is the mean arterial pressure. C may also be considered as equal to [1/(sysP + diaP)], where sysP and diaP are the measured systolic pressure and diastolic pressure, respectively. C may also be modeled as equal to [1/(MAP - const)], where const is a positive or negative constant number, and MAP is the mean arterial pressure. C may also be computed according to or based on a more complicated model, such as the Langewouters compliance, as described, for example, in Langewouters GJ, Wesseling KH, Goedhard WJ, The static elastic properties of 45 human thoracic and 20 abdominal aortas in vitro and the parameters of a new model, J Biomech. 1984; 17(6):425-35, where C is a function of gender and age, and nonlinearly depends on blood pressure.

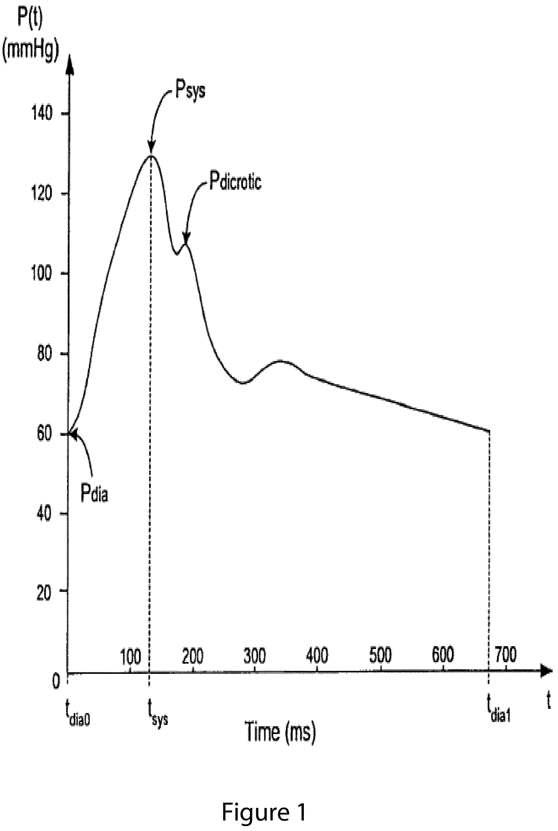

Figure 1 illustrates a measured blood pressure waveform over a single cardiac cycle, during which the blood pressure ranges between a diastolic pressure $P_{dia}$ and a systolic pressure $P_{sys}$.

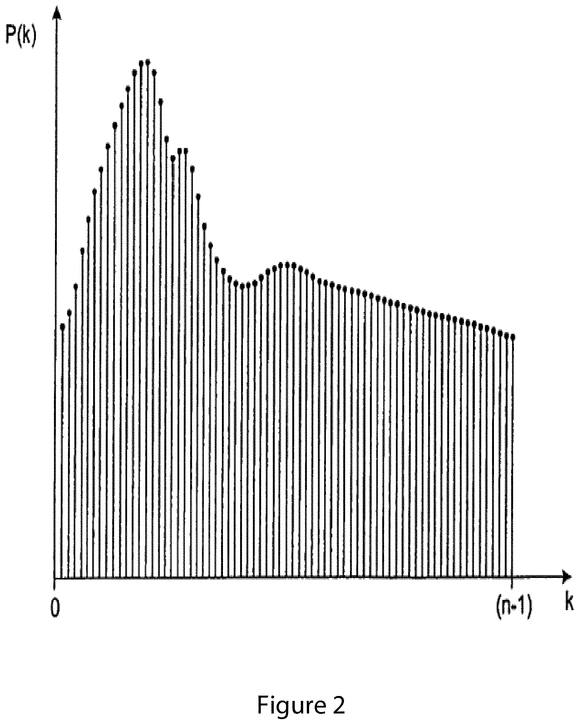

As suggested by Fig. 2, the analog blood pressure waveform of Fig. 1 can be digitized into a sequence of digital values using, *e.g.*, a standard analog-to-digital converter. This sequence of digital pressure values can be denoted in the form P(k), k=(0, n-1), where n is the number of sampled pressure values over the time and the portion of the pressure waveform selected for sampling and conversion. Note that the sampled portion of the pressure waveform may include a part of a single cardiac cycle, one entire cardiac cycle, or more than one cardiac cycle.

The sampled digital pressure values may then be grouped and treated statistically generally as described, *e.g.*, in United States Patent No. 7,967,757. Statistical moments including the mean, standard deviation, skewness and kurtosis may be computed corresponding to the selected sample set, P(k), k=(0, n-1).

In an ordered collection of m values, that is, a sequence Y(i), where i=0, ..., (m-1), the first four statistical moments $\mu_1$, $\mu_2$, $\mu_3$, and $\mu_4$, (*i.e.*, the statistical moments having orders of 1 through 4) of Y(i) can be calculated using well-known formulas. Specifically, $\mu_1$ is the mean (*i.e.*, the arithmetic average), $\mu_2 = \sigma^2$ is the variation (*i.e.*, the square of the standard deviation $\sigma$, $\mu_3$ is the skewness, and $\mu_4$ is the kurtosis. Thus:

$$\mu_1 = Y_{avg} = 1/m \times SUM(Y(i)) \qquad \text{(Formula 1)}$$

$$\mu_2 = \sigma^2 = 1/(m-1) \times SUM(Y(i) - Y_{avg})^2 \qquad \text{(Formula 2)}$$

$$\mu_3 = 1/(m-1) \times SUM\big[(Y(i) - Y_{avg})/\sigma\big]^3 \qquad \text{(Formula 3)}$$

$$\mu_4 = 1/(m-1) \times SUM\big[(Y(i) - Y_{avg})/\sigma\big]^4 \qquad \text{(Formula 4)}$$

In general, therefore, the β-th ordered moment $\mu_\beta$ can be expressed as:

$$\mu_\beta = 1/(m-1) \times 1/\sigma^\beta \times SUM\big[(Y(i) - Y_{avg})\big]^\beta$$

where i=0, ..., (m-1).

In some embodiments the first through fourth order moments of the pressure waveform P(k) (which can be denoted, e.g., as $\mu_{1P}$, $\mu_{2P}$, $\mu_{3P}$, and $\mu_{4P}$) are calculated and used in the computation of the first scaling factor K, where $\mu_{1P}$ is the mean, $\mu_{2P} = \sigma_P^2$ is the variation, that is, the square of the standard deviation $\sigma_P$, $\mu_{3P}$ is the skewness, and $\mu_{4P}$ is the kurtosis, where all of these moments are computed based on a series of sampled values from the pressure waveform P(k). The general formulas 1-4 above can be used to calculate these pressure-derived values after substituting P for Y, k for i, and n for m.

Formula 2 above provides the "textbook" method for computing a standard deviation. Other, more approximate methods might also be used. For example, at least in the context of blood pressure-based measurements, a rough approximation to $\sigma_P$, can be had by dividing by three the difference between the maximum and minimum measured pressure values, and that the maximum or absolute value of the minimum of the first derivative of the P(t) with respect to time is generally proportional to $\sigma_P$.

Some embodiments include computation of a scaling factor K not only as a function of moments of values corresponding directly to pressure values from the pressure waveform itself, P(k), but also of a pressure weighted time vector, the construction of which will be explained in further detail below.

As Fig. 2 indicates, at each discrete time k, the corresponding measured pressure will be P(k). The values k and P(k) can be formed into a sequence T(j) that corresponds to a histogram, in which each P(k) value is used as a "count" of the corresponding k value. By way of a greatly simplified example, assume that the entire pressure waveform consists of only four measured values P(1)=25, P(2)=50, P(3)=55, and P(4)=35. This sequence of measured pressure values could then be represented as a sequence T(j) with 25 ones, 50 twos, 55 threes, and 35 fours:
T(j)=1, 1, ..., 1, 2, 2, ... 2, 3, 3, ... 3, 4, 4, ... 4

This sequence would thus have 25+50+55+35=165 terms.

Statistical moments of varying orders can be computed for this sequence just as for any other. For example, the mean, (the first order statistical moment) is:

$$\mu_{1T} = (1*25+2*50+3*55+4*35)/165=430/165=2.606$$

The standard deviation $\sigma_T$ is the square root of the variation $\mu_{2T}$:

$$\sigma_T = \mathrm{SQRT}[1/164*(25(1\text{-}2.61)^2+50(2\text{-}2.61)^2+55(3\text{-}2.61)^2+35(4\text{-}2.61)^2)]=0.985$$

The skewness $\mu_{3T}$ and $\mu_{4T}$ can be computed by similar substitutions in Formulas 3 and 4, respectively:]

$$\mu_{3T} = \frac{1}{164} \times \left(\frac{1}{\sigma_T^3}\right) SUM[P(k) \times (k - \mu_{1T})^3]$$

$$\mu_{4T} = \frac{1}{164} \times \left(\frac{1}{\sigma_T^4}\right) SUM[P(k) \times (k - \mu_{1T})^4]$$

where k=1, ..., (m-1).

These calculations become very complex quite quickly, even with the obviously greatly simplified example given above, which includes only four sampled pressure values over the entire sampled interval. Any real-world implementation of these methods is very likely to sample many more data points over a given cardiac cycle or even a portion of it, and the associated computations would then be correspondingly more complex. This is clearly not something that could be performed in any meaningful way by a human alone, particularly in anything like real-time, as would likely be necessary for such methods to be of any practical therapeutic or diagnostic use. A computer processing system of adequate power and speed will thus be essential to the practice of methods like those described here, in any practical real-world application.

In practical applications, the blood pressure values may not be whole numbers. Depending on the resolution of the measurement and data storage apparatus, blood pressures might very well be measured and stored in whole number units corresponding, e.g., to millimeters of mercury (mm Hg). In such systems blood pressure values might be measured and stored, for example, in whole number units ranging between 0 and 200 mm Hg.

In other implementations the system resolution might be less. In some lower resolution implementations, for example, a pressure measured within 80-85 mm Hg might be treated as having an equivalent value, while pressures within 85-90 mm Hg might be treated as having an equivalent value of one higher "step". Various systems might thus assign the digitized pressure values to discrete pressure value "windows," with each such window reflecting a range of pressures of a size appropriate to the resolution of any given system.

As noted above, in some embodiments of the invention cardiac output can be calculated as

$$CO = C \times K \times \sigma \times PR.$$

In one implementation, K is computed as follows:

$$\begin{aligned}
K = {}&-145.44218 - 47.335793 \times v_1^{-2} \times v_3^2 \times v_{10} - 0.26164758 \\
&\times v_1^{-2} \times v_2^2 \times v_7^2 + 17.043701 \times v_1^{-1} \times v_2 \times v_7 \\
&- 11.336252 \times v_9^{-1} \times v_{10}^2 \times v_{11}^2 + 0.55703396 \times v_5^2 \times v_7^{-2} \\
&\times v_8^2 + 0.0021902458 \times v_5^2 \times v_6^2 \times v_7^2 - 69.638062 \times v_3^2 \\
&\times v_{10}^2 \times v_{11}^2 + 2943.7354 \times v_3^2 \times v_9^{-2} \times v_{10} + 147.5768 \\
&\times v_2 \times v_5^{-1} \times v_{11}^2 - 84.528252 \times v_2^2 \times v_4 \times v_5^{-2} \\
&- 0.1431479 \times v_1 \times v_2^{-1} \times v_5 + 218.56177 \times v_1 \times v_3^2 \\
&\times v_{10}
\end{aligned}$$

where, using the terminology related to the statistical moments of the pressure values and the pressure-weighted time series:

$v_1 = \sigma_P/10$, where $\sigma_P$ is the standard deviation of the sampled pressure values measured in mm Hg;

$v_2 = 600/HR$, where HR is the subject's heart rate, measured in beats per minute;

$v_3 = \mu_{1P}/100$, where $\mu_{1P}$ is the arithmetic mean of the sampled pressure values;

$v_4 = \sigma_T$, the standard deviation of the values in the pressure-weighted time series, derived as described above;

$v_5 = \mu_{1T}$, the arithmetic mean of the pressure-weighted time series;

$v_6 = \mu_{3P}$, the skewness of the sampled pressure values;

$v_7 = \mu_{4P}+3$, where $\mu_{4P}$ is the kurtosis of the sampled pressure values;

$v8 = \mu_{3T}$, the skewness of the pressure-weighted time series;

$v_9 = \mu_{4T}+3$, where $\mu_{4T}$ is the kurtosis of the pressure-weighted time series;

$$v_{10} = \frac{60/BSA \times B_1(sex)/(\pi \times B_2(sex))}{1+(\frac{\mu_{1P}-B_3(sex)}{B_2(sex)})^2}$$

$$v_{11} = BSA = 0.007184 \times (\text{subject height in cm})^{0.725} \times (\text{subject weight in kg})^{0.425}$$

where:

$B_i(sex)$ is element (i) of a respective array for the indicated sex of the subject, where specifically:

$$B_1(\text{male}) = 5.62;$$

$$B_2(\text{male}) = 57\text{-}0.44 \times (\text{subject age in years});$$

$$B_3(\text{male}) = 76\text{-}0.89 \times (\text{subject age in years});$$

$$B_1(\text{female}) = 4.12;$$

$$B_2(\text{female}) = 57\text{-}0.44 \times (\text{subject age in years});$$

and

$$B_3(\text{female}) = 72\text{-}0.89 \times (\text{subject age in years})$$

The example implementation described uses a set of sensed arterial pressure values for a substantial portion of the entire waveform over each cardiac cycle, at a sampling rate of 100 Hz. Other implementations might conceivably be based on waveforms over a portion of the cardiac cycle, or waveforms over more than one cardiac cycle, and different sampling rates might be used as well, depending on the particular implementation.

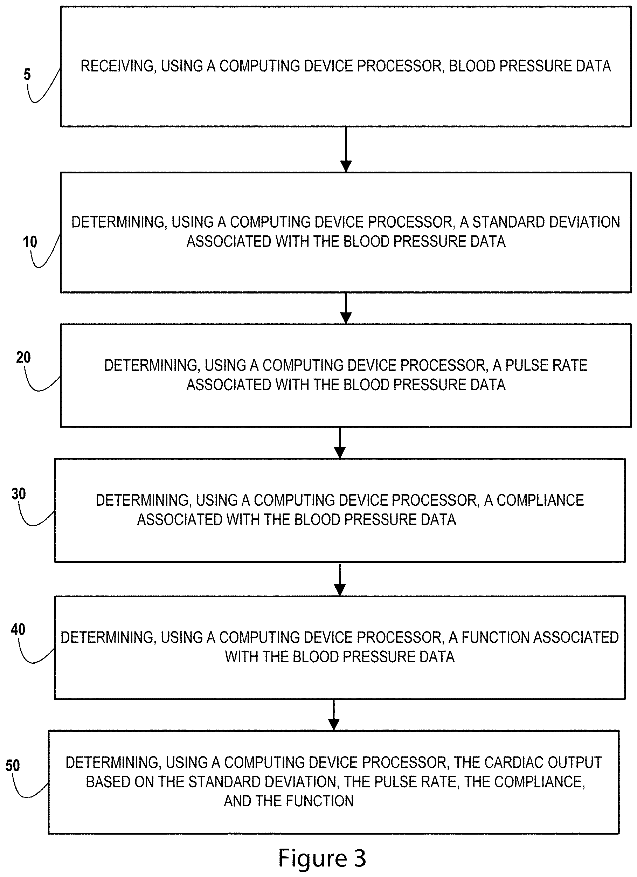

Figure 3 illustrates a process flow for a method for determining cardiac output. At block **5,** the process flow comprises receiving, using a computing device processor, blood pressure data. At block **10,** the process flow comprises determining, using a computing device processor, a standard deviation associated with the blood pressure data. At block **20,** the process flow comprises determining, using a computing device processor, a pulse rate associated with the blood pressure data. At block **30,** the process flow comprises determining, using a computing device processor, a compliance factor associated with the blood pressure data. At block **40,** the process flow comprises determining, using a computing device processor, a function associated with the blood pressure data. At block **50,** the process flow comprises determining, using a computing device processor, the cardiac output based on the standard deviation, the pulse rate, the compliance factor, and the function.

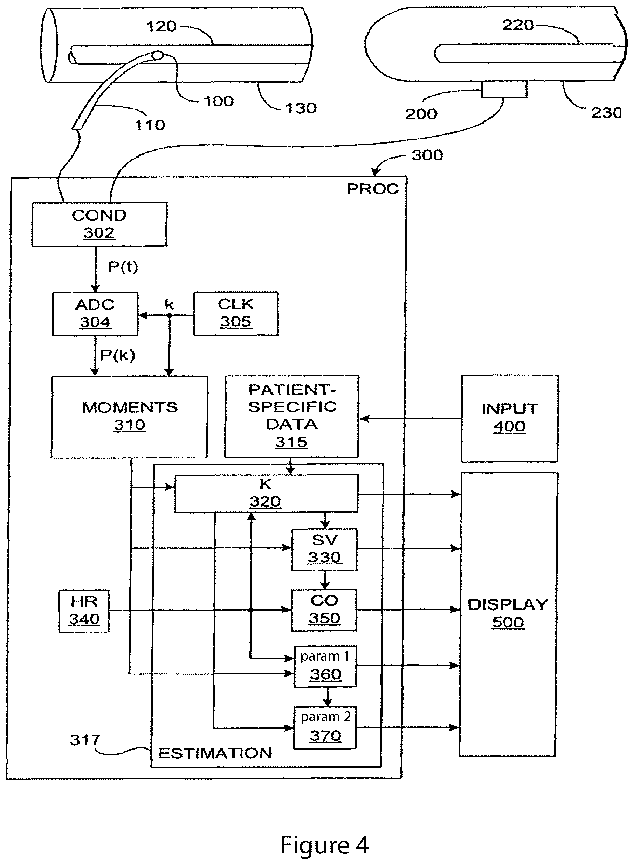

Figure 4 shows the main components of a system that implements the method described herein for sensing pressure and calculating a parameter such as a first scaling factor K and a second scaling factor C, SV, CO, etc. The system may be included within an existing patient-monitoring device, or it may be implemented as a dedicated monitor. Pressure, or some other input signal proportional to pressure, may be sensed either invasively, non-invasively, or both. Simply because it is anticipated to be the most common implementation of the disclosure, the system is described as measuring arterial blood pressure as opposed to some other input signal that corresponds to, that is indicative of, or that is converted to pressure.

Figure 4 shows both types of pressure sensing for the sake of conciseness; in most practical applications of the disclosure, either one or several variations will typically be implemented. In invasive applications of the disclosure, a conventional pressure sensor **100** is mounted on a catheter **110,** which is inserted in an artery **120** of a portion **130** of the body of a human or animal patient. Such an artery could be an ascending aorta, or pulmonary artery, or, in order to reduce the level of invasiveness, the artery **120** could be peripheral, such as the femoral, radial or brachial artery. In the non-invasive applications of the disclosure, a conventional pressure sensor **200,** such as a photo-plethysmographic blood pressure probe, is mounted externally in any conventional manner, for example using a cuff around a finger **230** or a transducer mounted on the wrist of the patient. In some embodiments, the sensor **100** or **200** may comprise a pressure transducer (e.g., a disposable pressure transducer DPT). Figure 4 schematically shows both types.

The signals from the sensors **100, 200** are passed via any known connectors as inputs to a processing system **300,** which includes one or more processors and other supporting hardware and system software (not shown) usually included to process signals and execute code. The disclosure may be implemented using a modified, standard, personal computer, or it may be incorporated into a larger, specialized monitoring system. In this disclosure, the processing system **300** also may include, or is connected to, conditioning circuitry **302** which performs such normal signal processing tasks as amplification, filtering, ranging, etc., as needed, as well as optional high pass filtering. The conditioned, sensed input pressure signal P(t) is then converted to digital form by a conventional analog-to-digital converter ADC **304,** which has or takes its time reference from a clock circuit **305.** As is well understood, the sampling frequency of the ADC **304** should be chosen with regard to the Nyquist criterion so as to avoid aliasing of the pressure signal. The output from the ADC **304** will be the discrete pressure signal P(k), whose values may be stored in conventional memory circuitry (not shown).

The values P(k) and the other required data and values are passed (usually, accessed from memory) to a software module **310** comprising computer-executable code for computing whichever of the parameters are to be used in the chosen algorithm for calculating the scaling factors C and K.

Patient-specific data such as age, height, weight, body surface area, etc., is stored in a memory **315,** which may also store other predetermined parameters. These values may be entered using any known input device **400** in the conventional manner.

An arterial compliance calculation module **320,** also comprising computer-executable code, then takes as inputs the various moments and patient-specific values and performs the chosen calculations for computing the first or the second scaling factors. For example, the module **320** could enter the parameters into the expression given below for the first or the second scaling factors, or into some other expression derived by creating a first or a second approximating function, respectively, that best fits a set of test data and provides as an output, the first or the second scaling factors. The calculation module **320** preferably also selects the time window over which each of the first or the second scaling factors, SV, CO, and other parameters or values (illustrated generically here as "param 1" and "param 2") are calculated, estimated, or otherwise generated by the system. This may be done as simply as choosing which and how many of the stored, consecutive, discretized P(t) values P(k) are used in each calculation, which is the same as selecting n in the range k=0, ..., (n-1).

Taking the scaling factors and other relevant parameters measured or otherwise provided to the system as inputs, a stroke volume computation module **330,** again comprising computer-executable code, then computes an SV estimate. Taking as inputs both SV and a heart rate value IIR generated by any known hardware device **340** or software routine (for example, using Fourier or derivative analysis) for measuring heart rate along with any other parameters described herein, a CO computation module **330** may then generate an estimate of CO using the method described herein.

Additional software modules **360** and **370** may be included to perform other calculations to estimate or derive other parameters or values of interest, which are suggested here by the non-specific referents "param 1" and "param 2" in the schematic diagram of Fig. 4.

As shown in Figure 4, the software modules **320, 330, 350, 360,** and **370,** that is, whichever of these are included, may be implemented within an estimation software component **317,** which may of course be combined with the moment-calculating component **310,** or with other software components of the processing system **300** as desired.

The disclosure further relates to a computer program loadable in a computer unit or the processing system **300** in order to execute the methods of the disclosure. Moreover, the various software modules **310, 315, 320, 330, 340, 350, 360,** and **370** used to perform the various calculations and perform related method steps according to the disclosure may also be stored as computer-executable instructions on a computer-readable medium in order to allow the instructions to be loaded into and executed by different processing systems. The functions executed by a software module described herein could additionally or alternatively be included in an application specific integrated circuit (ASIC), where the ASIC is specifically designed to execute the function.

In accordance with embodiments of the disclosure, the term "module" with respect to an apparatus may refer to a hardware component of the apparatus, a software component of the apparatus, or a component of the apparatus that includes both hardware and software. As used herein, a module may include one or more modules, where each module may reside in separate pieces of hardware or software. As used herein, an apparatus may alternatively be referred to as a "system" or a "device."

Although many embodiments of the present disclosure have just been described above, the present disclosure may be embodied in many different forms and should not be construed as limited to the embodiments set forth herein; rather, these embodiments are provided so that this disclosure will satisfy applicable legal requirements. Also, it will be understood that, where possible, any of the advantages, features, functions, devices, and/or operational aspects of any of the embodiments of the present disclosure described and/or contemplated herein may be included in any of the other embodiments of the present disclosure described and/or contemplated herein, and/or vice versa. In addition, where possible, any terms expressed in the singular form herein are meant to also include the plural form and/or vice versa, unless explicitly stated otherwise. Accordingly, the terms "a" and/or "an" shall mean "one or more," even though the phrase "one or more" is also used herein. Like numbers refer to like elements throughout.

As will be appreciated by one of ordinary skill in the art in view of this disclosure, the present disclosure may include and/or be embodied as an apparatus (including, for example, a system, apparatus, machine, device, computer program product, and/or the like), as a method (including, for example, a business method, computer-implemented process, and/or the like), or as any combination of the foregoing. Accordingly, embodiments of the present disclosure may take the form of an entirely business method embodiment, an entirely software embodiment (including firmware, resident software, micro-code, stored procedures in a database, or the like), an entirely hardware embodiment, or an embodiment combining business method, software, and hardware aspects that may generally be referred to herein as a "system" or "apparatus." Furthermore, embodiments of the present disclosure may take the form of a computer program product that includes a computer-readable storage medium having one or more computer-executable program code portions stored therein. As used herein, a processor, which may include one or more processors, may be "configured to" perform a certain function in a variety of ways, including, for example, by having one or more general-purpose circuits perform the function by executing one or more computer-executable program code portions embodied in a computer-readable medium, and/or by having one or more application-specific circuits perform the function.

It will be understood that any suitable computer-readable medium may be utilized. The computer-readable medium may include, but is not limited to, a non-transitory computer-readable medium, such as a tangible electronic, magnetic, optical, electromagnetic, infrared, and/or semiconductor system, device, and/or other apparatus. For example, in some embodiments, the non-transitory computer-readable medium includes a tangible medium such as a portable computer diskette, a hard disk, a random access memory (RAM), a read-only memory (ROM), an erasable programmable read-only memory (EPROM or Flash memory), a compact disc read-only memory (CD-ROM), and/or some other tangible optical and/or magnetic storage device. In other embodiments of the present disclosure, however, the computer-readable medium may be transitory, such as, for example, a propagation signal including computer-executable program code portions embodied therein.

One or more computer-executable program code portions for carrying out operations of the present disclosure may include object-oriented, scripted, and/or unscripted programming languages, such as, for example, Java, Perl, Smalltalk, C++, SAS, SQL, Python, Objective C, JavaScript, and/or the like. In some embodiments, the one or more computer-executable program code portions for carrying out operations of embodiments of the present disclosure are written in conventional procedural programming languages, such as the "C" programming languages and/or similar programming languages. The computer program code may alternatively or additionally be written in one or more multi-paradigm programming languages, such as, for example, F#.

Some embodiments of the present disclosure are described herein with reference to flowchart illustrations and/or block diagrams of apparatus and/or methods. It will be understood that each block included in the flowchart illustrations and/or block diagrams, and/or combinations of blocks included in the flowchart illustrations and/or block diagrams, may be implemented by one or more computer-executable program code portions. These one or more computer-executable program code portions may be provided to a processor of a general purpose computer, special purpose computer, and/or some other programmable data processing apparatus in order to produce a particular machine, such that the one or more computer-executable program code portions, which execute via the processor of the computer and/or other programmable data processing apparatus, create mechanisms for implementing the operations and/or functions represented by the flowchart(s) and/or block diagram block(s).

The one or more computer-executable program code portions may be stored in a transitory and/or non-transitory computer-readable medium (e.g., a memory or the like) that can direct, instruct, and/or cause a computer and/or other programmable data processing apparatus to function in a particular manner, such that the computer-executable program code portions stored in the computer-readable medium produce an article of manufacture including instruction mechanisms which implement the operations and/or functions specified in the flowchart(s) and/or block diagram block(s).

The one or more computer-executable program code portions may also be loaded onto a computer and/or other programmable data processing apparatus to cause a series of operational operations to be performed on the computer and/or other programmable apparatus. In some embodiments, this produces a computer-implemented process such that the one or more computer-executable program code portions which execute on the computer and/or other programmable apparatus provide operations to implement the operations specified in the flowchart(s) and/or the functions specified in the block diagram block(s). Alternatively, computer-implemented operations may be combined with, and/or replaced with, operator- and/or human-implemented operations in order to carry out an embodiment of the present disclosure.

While certain exemplary embodiments have been described and shown in the accompanying drawings, it is to be understood that such embodiments are merely illustrative of and not restrictive on the broad disclosure, and that this disclosure not be limited to the specific constructions and arrangements shown and described, since various other changes, combinations, omissions, modifications and substitutions, in addition to those set forth in the above paragraphs, are possible. Those skilled in the art will appreciate that various adaptations, modifications, and combinations of the just described embodiments can be configured without departing from the scope of the disclosure. Therefore, it is to be understood that, within the scope of the appended claims, the disclosure may be practiced other than as specifically described herein.

## Claims

1. A method for determining stroke volume (SV) of a subject, the method comprising:

   operating a processing system (300) to receive an input signal that corresponds to arterial blood pressure;
   operating the processing system (300) to calculate, as a first statistical moment, a standard deviation ($\sigma$) associated with numerical values corresponding to a sequence of measured arterial pressure values;
   operating the processing system (300) to calculate a function (K) of a second statistical moment associated with the numerical values corresponding to the sequence of measured arterial pressure values;
   **characterized by**
   operating the processing system (300) to calculate a compliance factor (C) which corresponds to the compliance of the subject's vasculature and is associated with the numerical values corresponding to the sequence of measured arterial pressure values;
   operating the processing system (300) to calculate the stroke volume (SV) as a function of the standard deviation ($\sigma$), the compliance factor (C) and the function (K), according to:

$$SV = C \times K \times \sigma;$$

   and
   operating a display to present an indication of the calculated stroke volume (SV) to a user.

2. The method of claim 1, wherein operating the processing system (300) to calculate the function (K) includes:

   operating the processing system (300) to calculate first through fourth order statistical moments ($\mu_{1P}$, $\mu_{2P}$, $\mu_{3P}$, $\mu_{4P}$) associated with the numerical values corresponding to the sequence of measured arterial pressure values; and
   using the first through fourth order statistical moments ($\mu_{1P}$, $\mu_{2P}$, $\mu_{3P}$, $\mu_{4P}$) for calculating the function (K).

3. The method of claim 1, wherein the compliance factor is inversely proportional to a mean of a measured arterial pressure of the subject.

4. The method of claim 1, wherein the compliance factor is inversely proportional to a mean of a measured arterial pressure of the subject minus a constant.

5. The method of claim 1, wherein the compliance factor is inversely proportional to at least one of the subject's measured systolic or diastolic pressure.

6. The method of claim 1, wherein the compliance factor is based on at least one of the subject's race, age, gender, or body surface area.

7. The method of claim 1, and further comprising:

   operating the processing system (300) to calculate a pulse rate (PR) associated with the numerical values corresponding to the sequence of measured arterial pressure values; and
   operating the processing system (300) to calculate the subject's cardiac output (CO) as the multiplication product of the stroke volume (SV) and the pulse rate (PR) according to:

$$CO = SV \times PR = C \times K \times \sigma \times PR.$$

8. The method of claim 2, wherein operating the processing system (300) to calculate a function (K) of numerical values corresponding to a sequence of measured arterial pressure values further includes:

   operating the processing system (300) to digitize an analog pressure signal to produce a sequence of digital values each of which corresponds to a arterial pressure value measured at a predetermined time;
   operating the processing system (300) to form a pressure-weighted sequence of values comprising counts of measured arterial pressure values within selected pressure value windows;
   operating the processing system (300) to calculate the first through fourth statistical moments ($\mu_{1T}$, $\mu_{2T}$, $\mu_{3T}$, $\mu_{4T}$) of the pressure-weighted sequence of values;
   wherein function (K) is calculated by further using first through fourth order statistical moments ($\mu_{1T}$, $\mu_{2T}$, $\mu_{3T}$, $\mu_{4T}$) of the pressure-weighted sequence of values.

9. An apparatus for determining a stroke volume (SV) of a subject, the apparatus comprising:

   means for receiving blood pressure data;
   means for determining, as a first statistical moment, a standard deviation ($\sigma$) associated with the blood pressure data;
   means for determining a function (K) of a second statistical moment associated with the blood pressure data;
   **characterized by**
   means for determining a compliance factor (C) which corresponds to the compliance of the subject's vasculature and is associated with the blood pressure data;
   means for determining the stroke volume (SV) based on the standard deviation ($\sigma$), the compliance factor (C), and the function (K) according to:

$$SV = C \times K \times \sigma.$$

10. The apparatus of claim 9, wherein the means for determining a function (K) uses first through fourth order statistical moments ($\mu_{1P}$, $\mu_{2P}$, $\mu_{3P}$, $\mu_{4P}$) associated with the numerical values corresponding to the sequence of measured arterial pressure values.

11. The apparatus of claim 9, further comprising:

   means for determining a pulse rate (PR) associated with the blood pressure data;
   means for calculating the subject's cardiac output (CO) as the multiplication product of the stroke volume (SV) and the pulse rate (PR) according to:

$$CO = SV \times PR = C \times K \times \sigma \times PR.$$

12. A computer program product comprising a computer-readable storage medium having one or more computer-executable program code portions stored therein, which, when executed by a processor, cause the processor to carry out the method of any of the claims 1 to 8.

**Patentansprüche**

1. Verfahren zum Bestimmen des Herzschlagvolumens (SV) eines Subjekts, wobei das Verfahren umfasst:

   Betreiben eines Verarbeitungssystems (300), um ein Eingangssignal zu empfangen, das dem arteriellen Blutdruck entspricht;
   Betreiben des Verarbeitungssystems (300), um als ein erstes statistisches Moment eine Standardabweichung ($\sigma$) zu berechnen, die numerischen Werten zugeordnet ist, die einer Sequenz von gemessenen arteriellen Druckwerten entsprechen;
   Betreiben des Verarbeitungssystems (300), um eine Funktion (K) eines zweiten statistischen Moments zu berechnen, das den numerischen Werten zugeordnet ist, die der Sequenz von gemessenen arteriellen Druckwerten entsprechen;
   **gekennzeichnet durch**
   Betreiben des Verarbeitungssystems (300), um einen Compliance-Faktor (C) zu berechnen, der der Compliance des Gefäßsystems des Subjekts entspricht und den numerischen Werten zugeordnet ist, die der Sequenz von gemessenen arteriellen Druckwerten entsprechen;
   Betreiben des Verarbeitungssystems (300), um das Herzschlagvolumen (SV) in Abhängigkeit von der Standardabweichung ($\sigma$), dem Compliance-Faktor (C) und der Funktion (K) zu berechnen gemäß:

$$SV = C \times K \times \sigma;$$

   und
   Betreiben eines Anzeigevorrichtung, um einem Benutzer eine Anzeige des berechneten Herzschlagvolumens (SV) zu präsentieren.

2. Verfahren nach Anspruch 1, wobei das Betreiben des Verarbeitungssystems (300) zum Berechnen der Funktion (K) enthält:

   Betreiben des Verarbeitungssystems (300), um statistische Momente erster bis vierter Ordnung ($\mu_{1P}$, $\mu_{2P}$, $\mu_{3P}$, $\mu_{4P}$) zu berechnen, die den numerischen Werten zugeordnet sind, die der Sequenz von gemessenen arteriellen Druckwerten entsprechen; und
   Verwenden der statistischen Momente erster bis vierter Ordnung ($\mu_{1P}$, $\mu_{2P}$, $\mu_{3P}$, $\mu_{4P}$) zum Berechnen der Funktion (K).

3. Verfahren nach Anspruch 1, wobei der Compliance-Faktor umgekehrt proportional zu einem Mittelwert eines gemessenen arteriellen Drucks des Subjekts ist.

4. Verfahren nach Anspruch 1, wobei der Compliance-Faktor umgekehrt proportional zu einem Mittelwert eines gemessenen arteriellen Drucks des Subjekts minus einer Konstante ist.

5. Verfahren nach Anspruch 1, wobei der Compliance-Faktor umgekehrt proportional zu wenigstens einem der gemessenen systolischen oder diastolischen Drücke des Subjekts ist.

6. Verfahren nach Anspruch 1, wobei der Compliance-Faktor auf wenigstens einem der Faktoren Rasse, Alter, Geschlecht oder Körperoberfläche des Subjekts basiert.

7. Verfahren nach Anspruch 1, und ferner umfassend:

   Betreiben des Verarbeitungssystems (300), um eine Pulsrate (PR) zu berechnen, die den numerischen Werten zugeordnet ist, die der Sequenz von gemessenen arteriellen Druckwerten entsprechen; und
   Betreiben des Verarbeitungssystems (300), um das Herzzeitvolumens (CO) des Subjekts als das Multiplikationsprodukt des Herzschlagvolumens (SV) und der Pulsrate (PR) zu berechnen gemäß:

$$CO = SV \times PR = C \times K \times \sigma \times PR.$$

8. Verfahren nach Anspruch 2, wobei das Betreiben des Verarbeitungssystems (300) zum Berechnen einer Funktion (K) von numerischen Werten, die einer Sequenz von gemessenen arteriellen Druckwerten entsprechen, ferner enthält:

Betreiben des Verarbeitungssystems (300), um ein analoges Drucksignal zu digitalisieren, um eine Sequenz von digitalen Werten zu erzeugen, von denen jeder einem zu einem vorbestimmten Zeitpunkt gemessenen arteriellen Druckwert entspricht;

Betreiben des Verarbeitungssystems (300), um eine druckgewichtete Sequenz von Werten zu bilden, die Zählungen von gemessenen arteriellen Druckwerten innerhalb ausgewählter Druckwertfenster umfassen;

Betreiben des Verarbeitungssystems (300), um die ersten bis vierten statistischen Momente ($\mu_{1T}$, $\mu_{2T}$, $\mu_{3T}$, $\mu_{4T}$) der druckgewichteten Sequenz von Werten zu berechnen;

wobei die Funktion (K) unter weiterer Verwendung der statistischen Momente erster bis vierter Ordnung ($\mu_{1T}$, $\mu_{2T}$, $\mu_{3T}$, $\mu_{4T}$) der druckgewichteten Sequenz von Werten berechnet wird.

9. Vorrichtung zum Bestimmen eines Herzschlagvolumens (SV) eines Subjekts, wobei die Vorrichtung umfasst:

Mittel zum Empfangen von Blutdruckdaten;

Mittel zum Bestimmen einer Standardabweichung ($\sigma$), die den Blutdruckdaten zugeordnet ist, als ein erstes statistisches Moment;

Mittel zum Bestimmen einer Funktion (K) eines zweiten statistischen Moments, das den Blutdruckdaten zugeordnet ist;

**gekennzeichnet durch**

Mittel zum Bestimmen eines Compliance-Faktors (C), der der Compliance des Gefäßsystems des Subjekts entspricht und den Blutdruckdaten zugeordnet ist;

Mittel zum Bestimmen des Herzschlagvolumens (SV) basierend auf der Standardabweichung ($\sigma$), dem Compliance-Faktor (C) und der Funktion (K) gemäß:

$$SV = C \times K \times \sigma.$$

10. Vorrichtung nach Anspruch 9, wobei die Mittel zum Bestimmen einer Funktion (K) statistische Momente erster bis vierter Ordnung ($\mu_{1P}$, $\mu_{2P}$, $\mu_{3P}$, $\mu_{4P}$) verwenden, die den numerischen Werten zugeordnet sind, die der Sequenz von gemessenen arteriellen Druckwerten entsprechen.

11. Vorrichtung nach Anspruch 9, ferner umfassend:

Mittel zum Bestimmen einer Pulsrate (PR), die den Blutdruckdaten zugeordnet ist;

Mittel zum Berechnen des Herzzeitvolumens (CO) des Subjekts als Multiplikationsprodukt des Herzschlagvolumens (SV) und der Pulsrate (PR) gemäß:

$$CO = SV \times PR = C \times K \times \sigma \times PR.$$

12. Computerprogrammprodukt, das ein computerlesbares Speichermedium umfasst, in dem ein oder mehrere von einem Computer ausführbare Programmcodeabschnitte gespeichert sind, die, wenn sie von einem Prozessor ausgeführt werden, den Prozessor veranlassen, das Verfahren nach einem der Ansprüche 1 bis 8 auszuführen.

**Revendications**

1. Procédé servant à déterminer le volume d'éjection systolique (SV) d'un patient, le procédé comprenant :

le fonctionnement d'un système de traitement (300) pour recevoir un signal d'entrée qui correspond à la pression artérielle ;

le fonctionnement du système de traitement (300) pour calculer, comme un premier moment statistique, un écart-type ($\sigma$) associé à des valeurs numériques correspondant à une séquence de valeurs de pression artérielle mesurées ;

le fonctionnement du système de traitement (300) pour calculer une fonction (K) d'un deuxième moment statistique associé aux valeurs numériques correspondant à la séquence de valeurs de pression artérielle mesurées ;

caractérisé en

le fonctionnement du système de traitement (300) pour calculer un facteur de conformité (C) qui correspond à la conformité du système vasculaire du patient et est associé aux valeurs numériques correspondant à la séquence de valeurs de pression artérielle mesurées ;

le fonctionnement du système de traitement (300) pour calculer le volume d'éjection systolique (SV) comme une fonction de l'écart-type (σ), du facteur de conformité (C) et de la fonction (K) selon :

$$SV = C \times K \times \sigma \; ;$$

et

le fonctionnement d'un écran permettant d'indiquer le volume d'éjection systolique (SV) à un utilisateur.

2. Procédé selon la revendication 1, dans lequel le fonctionnement du système de traitement (300) pour calculer la fonction (K) comporte :

le fonctionnement du système de traitement (300) pour calculer des moments statistiques du premier à quatrième ordre ($\mu_{1P}$, $\mu_{2P}$, $\mu_{3P}$, $\mu_{4P}$) associés aux valeurs numériques correspondant à la séquence de valeurs de pression artérielle mesurées ; et

l'utilisation des moments statistiques du premier à quatrième ordre ($\mu_{1P}$, $\mu_{2P}$, $\mu_{3P}$, $\mu_{4P}$) pour calculer la fonction (K).

3. Procédé selon la revendication 1, dans lequel le facteur de conformité est inversement proportionnel à une moyenne d'une pression artérielle mesurée du patient.

4. Procédé selon la revendication 1, dans lequel le facteur de conformité est inversement proportionnel à une moyenne d'une pression artérielle mesurée du patient moins une constante.

5. Procédé selon la revendication 1, dans lequel le facteur de conformité est inversement proportionnel à au moins une de la pression systolique ou diastolique mesurée du patient.

6. Procédé selon la revendication 1, dans lequel le facteur de conformité est basé sur au moins un du genre, de l'âge, de la race ou de la surface corporelle du patient.

7. Procédé selon la revendication 1 et comprenant en outre :

le fonctionnement du système de traitement (300) pour calculer une fréquence du pouls (PR) associée aux valeurs numériques correspondant à la séquence de valeurs de pression artérielle mesurées ; et

le fonctionnement du système de traitement (300) pour calculer le débit cardiaque (CO) du patient comme le produit de la multiplication du volume d'éjection systolique (SV) avec la fréquence du pouls (PR) selon :

$$DC = VES \times FP = C \times K \times \sigma \times FP.$$

8. Procédé selon la revendication 2, dans lequel le fonctionnement du système de traitement (300) pour calculer une fonction (K) de valeurs numériques correspondant à une séquence de valeurs de pression artérielle mesurées comporte en outre :

le fonctionnement du système de traitement (300) pour numériser un signal de pression analogique afin de produire une séquence de valeurs numériques dont chacune correspond à une valeur de pression artérielle mesurée à un temps prédéterminé ;

le fonctionnement du système de traitement (300) pour obtenir une séquence de pression pondérée de valeurs comprenant des comptes de valeurs de pression artérielle mesurées à l'intérieur de fenêtres de valeurs de pression sélectionnées ;

le fonctionnement du système de traitement (300) pour calculer les moments statistiques du premier à quatrième ordre ($\mu_{1T}$, $\mu_{2T}$, $\mu_{3T}$, $\mu_{4T}$) de la séquence de pression pondérée de valeurs ;

dans lequel la fonction (K) est calculée en continuant à utiliser les moments statistiques du premier à quatrième ordre ($\mu_{1T}$, $\mu_{2T}$, $\mu_{3T}$, $\mu_{4T}$) de la séquence de pression pondérée de valeurs.

9. Appareil servant à déterminer un volume d'éjection systolique (SV) d'un patient, l'appareil comprenant :

des moyens pour recevoir des données de pression artérielle ;
des moyens pour déterminer, comme un premier moment statistique, un écart-type ($\sigma$) associé aux données de pression artérielle ;
des moyens pour déterminer une fonction (K) d'un deuxième moment statistique associé aux données de pression artérielle ;
caractérisé en
des moyens pour déterminer un facteur de conformité (C) qui correspond à la conformité du système vasculaire du patient et est associé aux données de pression artérielle ;
des moyens pour déterminer le volume d'éjection systolique (SV) basé sur l'écart-type ($\sigma$), le facteur de conformité (C) et la fonction (K) selon :

$$VES = C \times K \times \sigma.$$

10. Appareil selon la revendication 9, dans lequel les moyens pour déterminer une fonction (K) utilisent des moments statistiques du premier à quatrième ordre ($\mu_{1P}$, $\mu_{2P}$, $\mu_{3P}$, $\mu_{4P}$) associés aux valeurs numériques correspondant à la séquence de valeurs de pression artérielle mesurées.

11. Appareil selon la revendication 9 comprenant en outre :

des moyens pour déterminer une fréquence du pouls (PR) associée aux données de pression artérielle ;
des moyens pour calculer le débit cardiaque (CO) du patient comme le produit de la multiplication du volume d'éjection systolique (SV) avec la fréquence du pouls (PR) selon :

$$DC = VES \times FP = C \times K \times \sigma \times FP.$$

12. Progiciel comprenant un support mémoire lisible par ordinateur ayant une ou plusieurs parties de code de programme réalisable par ordinateur stockées dans celui-ci qui, si exécutées par un processeur, amènent le processeur à réaliser le procédé selon l'une quelconque des revendications 1 à 8.

Figure 1

Figure 2

RECEIVING, USING A COMPUTING DEVICE PROCESSOR, BLOOD PRESSURE DATA

DETERMINING, USING A COMPUTING DEVICE PROCESSOR, A STANDARD DEVIATION ASSOCIATED WITH THE BLOOD PRESSURE DATA

DETERMINING, USING A COMPUTING DEVICE PROCESSOR, A PULSE RATE ASSOCIATED WITH THE BLOOD PRESSURE DATA

DETERMINING, USING A COMPUTING DEVICE PROCESSOR, A COMPLIANCE ASSOCIATED WITH THE BLOOD PRESSURE DATA

DETERMINING, USING A COMPUTING DEVICE PROCESSOR, A FUNCTION ASSOCIATED WITH THE BLOOD PRESSURE DATA

DETERMINING, USING A COMPUTING DEVICE PROCESSOR, THE CARDIAC OUTPUT BASED ON THE STANDARD DEVIATION, THE PULSE RATE, THE COMPLIANCE, AND THE FUNCTION

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110275943 A1
- US 4236527 A, Newbower
- US 4507974 A, Yelderman
- US 5146414 A, McKown
- US 5687733 A, McKown
- US 5400793 A, Wesseling
- US 5535753 A, Petrucelli
- US 7967757 B
- US 8721556 B
- WO 2010091055 A

**Non-patent literature cited in the description**

- **LANGEWOUTERS GJ ; WESSELING KH ; GOED-HARD WJ.** The static elastic properties of 45 human thoracic and 20 abdominal aortas in vitro and the parameters of a new model. *J Biomech.,* 1984, vol. 17 (6), 425-35